# EUROPÄISCHE PATENTSCHRIFT

(11) **EP 1 642 568 B1**
(45) Veröffentlichungstag und Bekanntmachung des Hinweises auf die Patenterteilung: **09.06.2010**
(21) Anmeldenummer: 05016851.7
(22) Anmeldetag: 02.08.2005
(51) Int. Cl.: A61K 8/64, A61K 8/34, A61K 8/46, A61Q 11/00

(54) **Mundspüllösung**
Mouthwash
Solution de rinçage dentaire

(30) Priorität: 03.08.2004 DE 102004037598
(43) Veröffentlichungstag der Anmeldung: 05.04.2006
(73) Patentinhaber: Prontomed GmbH, 32120 Hiddenhausen (DE)
(72) Erfinder: Widl, Hans F., 32120 Hiddenhausen (DE); Dahlen, Neithard, 35510 Butzbach (DE)
(74) Vertreter: Tomerius, Isabel

(56) Entgegenhaltungen:
- WO-A-02/38161
- WO-A-93/21903
- WO-A-94/16674
- US-A1- 2001 006 647

## Beschreibung

Die Erfindung betrifft ein Mittel zur Anwendung im Mund- und Rachenraum, welches in wässriger Lösung als Mikrobizid ein lineares polymeres Biguanid und/oder ein wasserlösliches Salz desselben enthält,
**dadurch gekennzeichnet,**
**dass** es das Mikrobizid in Kombination mit wenigstens einem Süßstoff umfasst. Die Verwendung des Mittels ist zur Spülung des Mund- und Rachenraums zur Beseitigung unangenehmer Gerüche und Ausdünstungen geeignet.

Im Stand der Technik, wie beispielsweise aus der WO 94/16674 A1, sind zur Desinfektion der Mundhöhle wässrige Lösungen bekannt, die als mikrobiziden Wirkstoff und zur Konservierung der Lösung Alkohole enthalten. Anstelle der Alkohole wird als Wirkstoff häufig auch ein Chlorhexidin-Derivat wie beispielsweise Chlorhexidindigluconat eingesetzt. Vor allem in Lösungen, die bei Entzündungen im Mund- und Rachenraum eingesetzt werden, ist häufig Chlorhexidin enthalten.

Chlorhexidin hat jedoch den Nachteil, dass es wie viele andere mikrobizide Wirkstoffe auch zytotoxisch auf die menschliche Gewebszelle wirkt. Die mikrobizide Wirkung von Chlorhexidin ist ferner bei gramnegativen Bakterien nicht besonders gut ausgeprägt. Außerdem führt Chlorhexidin zu einer verstärkten Zahnsteinbildung und, bei längerer Anwendung, zu einer Verfärbung von Zähnen, Zahnfüllungen und der Zungenoberfläche. Schließlich kann Chlorhexidin die Geschmacksempfindung irritieren und in seltenen Fällen Schleimhautbrennen verursachen.

Hexetidin weist den Nachteil auf, dass es in wässrigen Lösungen schwer löslich ist. Es stellt somit eine nicht zu unterschätzende Herausforderung dar, Hexetidin in eine akzeptable galenische Form zu überführen, die dann auch noch ausreichend wirksam ist. Tatsächlich kann beobachtet werden, dass eine Reihe bekannter Hexetidin-rialtiger Lösungen nur eine unzureichende antimikrobielle Wirksamkeit aufweist, die nicht an die Wirksamkeit Chlorhexidin-haltiger Präparate heranreicht.

Es bestand daher ein Bedarf an einem Mittel zur Anwendung im Mund- und Rachenraum, das auch bei langzeitiger Anwendung eine ausgeprägte mikrobizide Wirkung auf alle relevanten, im Mund- und Rachenraum vorkommenden Keime besitzt, dabei aber die oben genannten Nachteile nicht aufweist. **Aufgabe** der Erfindung ist es, ein derartiges Mittel zur Verfügung zu stellen.

Die Lösung dieser Aufgabe gelingt mit dem Mittel gemäß Anspruch 1. Bevorzugte Ausführungsformen sind in den Unteransprüchen beschrieben.

Die Erfindung bezieht sich also auf ein Mittel zur Anwendung im Mund- und. Rachenraum, welches in wässriger Lösung als Mikrobizid ein lineares polymeres Biguanid und/oder ein wasserlösliches Salz des selben enthält. Zusätzlich zum Biguanid ist außerdem wenigstens ein Süßstoff und/oder Zuckeraustauschstoff enthalten.

Im Rahmen der Erfindung eingesetzt werden können alle linearen polymeren Biguanide, die eine keimtötende Wirkung besitzen. Solche linearen polymeren Biguanide werden beispielsweise auf dem Gebiet ophtalmologischer Lösungen oder bei kosmetischen Produkten als mikrobizide Wirkstoffe eingesetzt und sind dort aus zum Beispiel den Druckschriften WO93/2190 A1, WO 02/38161 A1 und US 2001/0006647 A1 bekannt. Bevorzugtes Biguanid ist erfindungsgemäß jedoch Polyhexamethylenbiguanid (PHMB). Wie alle übrigen Biguanide kann es als solches oder in Form eines wasserlöslichen Salzes eingesetzt werden. Bevorzugt ist hier das Hydrochlorid, das beispielsweise in Form einer 20-prozentigen wässrigen Lösung unter der Bezeichnung Vantocil^{®} IB von der Firma Arch Chemicals, Manchester, CB, erhältlich ist. Wenn im folgenden allgemein von einem Biguanid oder PHMB die Rede ist, sollen gleichzeitig immer auch die Salze dieser Verbindungen mit angesprochen sein. Die Molekulargewichtsbereiche, in denen die Biguanide eingesetzt werden können, sind nicht besonders beschränkt. Vielmehr können alle diese Verbindungen mit den bislang üblichen Molekulargewichten verwendet werden. Im Falle von PHMB liegt das Molekulargewicht beispielsweise in einem Bereich von etwa 1500 bis 15000. Bevorzugt sind Molekulargewichte des PHMB unter 5000 und insbesondere unter 2900.

Im Stand der Technik ist PHMB bisher vor allem als Wundbehandlungsmittel bekannt. So wird in der DE-A-10012026 der Anmelderin die Anwendung eines wässrigen, PHMB-haltigen Gels zum Lösen von Verkrustungen und Borken sowie die Verwendung beim feuchten Wundwechsel beschrieben. In der DE-A-1 0132817 der Anmelderin wird ein Wundbehandlungsmittel offenbart, das zum Beispiel in Form eines Wasch- oder Duschgels zur Dekontamination der Haut benutzt werden kann. Das Wundbehandlungsmittel enthält in wässriger Lösung Polyhexamethylenbiguanid in Kombination mit einem Tensid, das aus einem Glycin-Derivat, einem, Sulfosuccinat oder einem Amid auf Basis einer Fettsäure ausgewählt ist. Bevorzugte Tenside sind Amidoalkylbetaine wie beispielsweise Undecylensäureamidopropylbetain. Das Wundbehandlungsmittel hat sich zur Dekontamination besonders von chronischen Wunden bewährt, da der Wirkstoff PHMB gegen praktisch alle klinisch relevanten Krankheitserreger wirksam ist, dabei aber das sehr empfindliche Wundgewebe nicht schädigt. Durch den Zusatz des Tensids werden Wundbelege besonders wirksam entfernt, wodurch sich die Wundheilung erheblich beschleunigt.

Aufgrund seines breiten Wirkungsspektrums erscheint Polyhexamethylenbiguanid grundsätzlich auch für den Einsatz in Mundspüllösungen geeignet. Dem steht jedoch entgegen, dass PHMB einen ausgeprägten und alles andere als angenehmen Eigengeschmack besitzt. Um überhaupt einen Einsatz als Mund- und Rachenspüllösung zu erlauben, war es daher erforderlich, den Eigengeschmack des PHMB zu kaschieren. Dabei zeigte es sich jedoch, dass eine Vielzahl der üblicherweise in Mundspüllösungen eingesetzten Geschmacks- und Aromastoffe die Wirksamkeit des Biguanids drastisch herabsetzten oder ganz zunichte machten. So führte beispielsweise der Zusatz von Tee-Extrakten, vielen ätherischen Ölen und Zucker zu einem starken und teilweise vollständigen Wirkungsverlust des PHMB. Die Ursachen hierfür sind nicht vollständig klar. Es wird jedoch vermutet, dass die mikrobizide Wirksamkeit des Biguanids durch elektrolytische Spannung hervorgerufen wird, die zwischen dem Biguanid und der anionisch aufgeladenen bakteriellen Zellmembran entsteht. Diese elektrolytische Spannung scheint durch den Zusatz von geschmackskaschierenden Substanzen gestört zu werden, wodurch die mikrobizide Wirksamkeit des Biguanids verloren geht.

Die Erfindung beruht auf der Erkenntnis, dass durch den Zusatz von Süßstoff undeventuell Zuckeraustauschstoff der beschriebene Wirksamkeitsverlust bei linearen polymeren Biguaniden wie PHMB nicht oder nur in geringem Maße eintritt. Bei den Süßstoffen handelt es sich um synthetische oder natürliche Verbindungen mit einem intensiv süßen Geschmack. Sie zählen zu den Lebensmittelzusatzstoffen. Zuckeraustauschstoffe sind Polyole, die ebenfalls süß schmecken, aber weniger ausgeprägt als Süßstoffe. Sie besitzen einen etwas geringeren Nährwert als Zucker, aber mehr Kalorien als Süßstoffe, die keinen oder nur einen vernachlässigbar geringen Nährwert aufweisen. Süßstoffe werden vom Körper völlig oder weitgehend unverändert ausgeschieden. Von Vorteil ist weiterhin, dass Süßstoffe und einige, erfindungsgemäß entsprechend bevorzugte, Zuckeraustauschstoffe die Entstehung von Karies nicht begünstigen.

Im Rahmen der Erfindung geeignete Süßstoffe sind die Süßstoffgruppen Acesulfam, Aspartam, Cyclamat, Neohesperidin, Saccharin und Thaumatin. Unter diesen sind Acesulfam (E 950) und Aspartam (E 951) aufgrund des nicht optimalen Geschmacks des resultierenden Mittels zur Anwendung im Mund- und Rachenraum weniger bevorzugt. Thaumatin (E 957) ist wegen seines hohen Preises weniger geeignet. Gut geeignet sind dagegen Saccharin (E 954), Neohesperidin (E 959) und Cyclamat (E 952). Cyclamat bezeichnet eine Gruppe von Süßstoffen, die Cyclohexansulfamidsäure und deren Natrium-und Kaliumsalze umfasst. Die Süßkraft von Cyclamat ist 35 bis 70 mal höher als die von Haushaltszucker. Cyclamat und insbesondere das erfindungsgemäß bevorzugte Natriumcyclamat sind aufgrund ihrer hohen Süßkraft geeignet, den unangenehmen Eigengeschmack von PHMB zu überdecken. Dies gelingt ebenfalls gut mit dem Süßstoff Neohesperidin, der durch chemische Synthese aus Flavonoiden hergestellt wird. Durch den leicht mentholhaltigen Eigengeschmack eignen sich Neohesperidin-Süßstoffe sehr gut zur Anwendung in Mund- und Rachenspüllösungen.

Zu den Zuckeraustauschstoffen zählen Mannitol (E 421), Sorbitol (E 420), lsomaltol (E 953), Maltitol (E 965), Lactitol (E 966) und Xylitol (E 967). Erfindungsgemäß bevorzugte Zuckeraustauschstoffe sind Xylitol und Sorbitol, die auch in Kombination miteinander eingesetzt werden können.

Als besonders günstig hat es sich erwiesen, eine Kombination mehrerer Süßstoffe und Zuckeraustauschstoffe einzusetzen. Süßstoffe und Zuckeraustauschstoffe, die insgesamt hier als Süßungsmittel bezeichnet werden, schmecken jeweils unterschiedlich intensiv und unterschiedlich lange süß. Während bei einigen der Süßungsmittel die Geschmacksentwicklung unmittelbar einsetzt, dafür aber rasch abklingt, ist es bei anderen genau umgekehrt. Eine geeignete Kombination "schnell schmeckender" mit "lang schmeckenden" Süßungsmitteln kann daher im Ergebnis ein rasch einsetzendes und lang anhaltendes Geschmacksempfinden hervorrufen. Zudem gelingt es durch geeignete Auswahl der miteinander kombinierten Süßungsmittel deren Konzentration im Mundbehandlungsmittel insgesamt zu reduzieren. Damit verringert sich auch die Gefahr, das lineare polymere Biguanid in seiner Wirkung zu inaktivieren.

Besonders bevorzugt ist es, wenn das Süßungsmittel wenigstens einen Süßstoff enthält, der in Kombination mit wenigstens einem anderen Süßstoff und/oder wenigstens einem anderen Zuckeraustauschstoff eingesetzt wird. Als bevorzugter Süßstoff wird dabei ein Cyclamat eingesetzt. Zweckmäßig wird Cyclamat in Kombination mit wenigstens einem der Süßungsmittel Saccharin, Acesulfam, Aspartam, Xylitol oder Sorbitol verwendet. Geeignet sind beispielsweise die Kombinationen Cyclamat + Saccharin, Cyclamat + Acesulfam + Aspartam, Cyclamat + Xylitol oder Cyclamat + Sorbitol. Dabei kann das Gewichtsverhältnis von Cyclamat zu den jeweiligen weiteren Süßungsmitteln der Kombination im Allgemeinen im Bereich von 100 : 1 bis 1 : 100 liegen. Bevorzugte Gewichtsanteile liegen im Falle von Cyclamat + Saccharin bei 1 : 1 bis 20 : 1, von Cyclamat + (Acesulfam + Aspartam) bei 50 : 1 bis 1 : 1, speziell bei 20 : 0,5 : 1, von Cyclamat + Xylitol bei 1 : 50 bis 1 : 10 oder von Cyclamat + Sorbitol bei 1 : 50 bis 1 : 10.

Eine geeignete Kombination ist auch Xylitol + Saccharin, zweckmäßig in Gewichtsanteilen im Bereich von 200 : 1 bis 20 : 1.

Im erfindungsgemäßen Mundbehandlungsmittel kann zudem wenigstens ein Polyol vorhanden sein, wobei unter diesem Begriff nicht die als Zuckeraustauschstoffe bezeichneten Polyole gemeint sind. Unter diesen Polyolen sind insbesondere Glycerin (E 422), Propylenglykol (E 1520) und Polyethylenglykol zu verstehen. Unter den Polyethylenglykolen sind solche mit einem Molekulargewicht von 400 bis 4000 bevorzugt. Die genannten Polyole haben ebenfalls einen süßlichen Eigengeschmack. Sie eignen sich daher ebenfalls, in Kombination mit den genannten Süßstoffen und/oder Zuckeraustauschstoffen eingesetzt zu werden. Besonders geeignet ist dabei erneut die Kombination mit Cyclamat, wobei sich besonders eine Mischung von Cyclamat mit Glycerin anbietet, zweckmäßig in einem Gewichtsverhältnis von 1 : 100 bis 100 : 1, bevorzugt von 1 : 50 bis 1 : 20.

Die Menge des Süßstoffes in dem erfindungsgemäßen Mittel hängt einerseits von der Art des Süßstoffs selbst ab sowie von der Art und Konzentration des linearen polymeren Biguanids im erfindungsgemäßen Mittel zur Anwendung im Mund- und Rachenraum. Gleiches gilt für die Zuckeraustauschstoffe. Die Konzentration kann leicht durch einfache Versuche ermittelt werden und wird zweckmäßig so gewählt, dass einerseits der unangenehme Geschmack des Biguanids ausreichend kaschiert wird, andererseits der Verlust der mikrobiziden Wirkung, wenn ein solcher denn überhaupt durch den Zusatz des gewählten Süßstoffs und/oder Zuckeraustauschstoffes beobachtet wird, möglichst gering ist. Im Allgemeinen wird der Süßstoff in einer Konzentration von 0,01 bis 1 Gew.-%, bevorzugt 0,02 bis 0,5 Gew.-%, in der wässrigen Lösung enthalten sein. Besonders geeignete Mengen liegen bei 25 bis 2500 mg/l der wässrigen Lösung. Der Zuckeraustauschstoff ist üblicherweise in einer Konzentration von 0,2 bis 10 Gew.-%, bevorzugt 1,0 bis 5,0 Gew.-%, in der wässrigen Lösung enthalten. Gewisse Konzentrationsänderungen können sich gegebenenfalls ergeben, wenn in dem erfindungsgemäßen Mittel weitere Komponenten vorhanden sind, die eine etwas andere Dosierung des Süßstoffs und/oder Zuckeraustauschstoffes zweckmäßig erscheinen lassen.

Zu den weiteren Komponenten können, neben den bereits erwähnten Polyolen, beispielsweise ätherische Öle zählen. Diese können zur weiteren Verbesserung des Geschmacks des erfindungsgemä-ßen Mittels zugefügt werden. Hier sind grundsätzlich alle solchen ätherischen Öle denkbar, die bereits bisher in Mund- oder Rachenspüllösungen eingesetzt wurden, solange sie nicht zu einem Verlust der Wirksamkeit des polymeren linearen Biguanids führen. Einige ätherische Öle weisen selbst eine gewisse antibakterielle und entzündungshemmende Wirkung auf. Der Zusatz eines ätherischen Öls kann sich daher wirkungsverstärkend im erfindungsgemäßen Mittel auswirken. Besonders bevorzugt ist es, wenn das ätherische Öl ausgewählt ist aus wenigstens einem Öl der Gruppe Pfefferminzöl (Arzneibuchvorschrift: EB 6), Eukalyptusöl (Ph. Eur.), Zitronenöl (Ph. Eur.), Orangenöl, Grapefruitöl, Thymianöl (DAC) und Kamillenöl (DAB 2000). Unter diesen ist momentan eine Mischung von Pfefferminz- und Eukalyptusöl besonders bevorzugt. Geeignete Mengen des ätherischen Öls liegen üblicherweise bei 0,3 bis 3 Gew.-%, bevorzugt 0,8 bis 3 Gew.-%, bezogen auf die anwendungsfertige wässrige Lösung. Bei Bedarf kann das ätherische Öl zusätzlich mit weiteren, dem Fachmann bekannten synthetischen und/oder naturidentischen Aromastoffen ergänzt werden.

Um das ätherische Öl in die wässrige Lösung einzubinden, wird dieses bevorzugt kolloidal solubilisiert. Hierzu wird zweckmäßig wenigstens ein Tensid zugesetzt. Geeignet sind hier insbesondere nicht ionische und/oder Amphotenside, da diese die mikrobizide Wirksamkeit des zugefügten linearen polymeren Biguanids wenig oder gar nicht beeinträchtigen. Da die angesprochenen Tenside oft einen wenig angenehmen Geschmack aufweisen, sollte ihre Konzentration im erfindungsgemäßen Mittel möglichst gering gehalten werden. Zweckmäßig wird nur gerade soviel Tensid zugefügt, wie zur Solubilisierung des ätherischen Öls erforderlich ist. In der Regel werden etwa 1 bis 10 Gew.-% Tensid, bezogen auf die wässrige Lösung, verwendet. Gegebenenfalls kann die Konzentration des zugefügten Süßstoffs erhöht werden, um den Eigengeschmack des Tensids zu kaschieren. Für die Solubilisierung der ätherischen Öle haben sich Tenside besonders bewährt, die ausgewählt sind aus Macrogol-Tensiden und/oder Polysorbat. Bevorzugte Macrogol-Tenside sind ausgewählt aus Macrogolglycerolhydroxylalkylat und Macrogolalkylether, wobei Alkyl C₈₋₂₂-Alkyl und insbesondere C₁₂₋₁₈-Alkyl ist. Unter diesen sind Macrogolglycerolhydroxylstearat (zum Beispiel Cremophor® RH40) und Macrogollaurylether (Mulsifan®, Brij® oder Dehydol®) besonders bevorzugt. Bevorzugtes Polysorbat ist Polysorbat 80 (zum Beispiel Tween® 80). Bei den meisten dieser Tenside handelt es sich um flüssige Tenside, die kalt oder nur bei leichter Erwärmung mit der wässrigen Lösung gemischt werden können, um das zugesetzte ätherische Öl in eine klare oder höchstens opaleszierende wässrige Lösung zu überführen. Auch feste Tenside können jedoch grundsätzlich verwendet werden.

Zusätzlich oder alternativ zu den genannten Solubilisierungstensiden kann das erfindungsgemäße Mittel wenigstens ein weiteres Tensid enthalten, bei dem es sich um ein Glycin-Derivat und insbesondere ein Amidoalkylbetain einer Fettsäure handelt. Die Kombination derartiger Tenside mit linearen polymeren Biguaniden ist grundsätzlich bereits in der schon eingangs erwähnten DE-A-10132817 der Anmelderin beschrieben. Es kann daher auf die Offenbarung dieser Druckschrift Bezug genommen werden. Bevorzugte Glycinderivat-Tenside sind Undecylenamidoalkylbetain, Cocamidoalkylbetain, Lauramidoalkylbetain oder Ricinolamidoalkylbetain, wobei der Alkylrest bevorzugt Ethyl oder Propyl ist. Unter diesen besonders bevorzugt ist Undecylensäureamidopropylbetain. Die Konzentration des Glycinderivat-Tensids richtet sich einerseits nach der Art des Tensids selbst, andererseits nach den verwendeten anderen Komponenten des erfindungsgemäßen Mittels. Geeignete Konzentrationen liegen insbesondere für das bevorzugte Undecylensäureamidopropylbetain bei Konzentrationen von 0,01 bis 1,5 Gewichtsprozent, insbesondere 0,03 bis 1 Gewichtsprozent und bevorzugt 0,1 bis 0,4 Gewichtsprozent.

Auch die Konzentration des mikrobiziden Wirkstoffs selbst, des linearen polymeren Biguanids, hängt im Wesentlichen von den verwendeten weiteren Komponenten im erfindungsgemäßen Mittel zur Anwendung im Mund- und Rachenraum sowie von der beabsichtigten Indikation ab. Es werden die für die jeweilige Indikation geeigneten medizinisch oder therapeutisch wirksamen Konzentrationen eingesetzt. Sind im erfindungsgemäßen Mittel Komponenten vorhanden, welche die Wirksamkeit des linearen polymeren Biguanids beeinträchtigen, wird die Konzentration des letzteren zweckmäßig entsprechend erhöht, um den Wirkungsverlust auszugleichen. Geeignete Konzentrationen liegen insbesondere für das bevorzugte PHMB im Bereich von 0,01 bis 0,3 Gewichtsprozent, insbesondere bei etwa 0,1 Gewichtsprozent, der anwendungsfertigen Lösung.

Zusätzlich zu den genannten Komponenten kann das erfindungsgemäße Mittel weitere Substanzen enthalten. Grundsätzlich können dies beispielsweise solche Substanzen sein, die bereits bisher in Mitteln zur Behandlung des Mund- oder Rachenraumes eingesetzt wurden. Diese Substanzen sollten aber mit den anderen Komponenten des erfindungsgemäßen Mittels kompatibel sein und insbesondere die mikrobizide Wirksamkeit des Biguanids gar nicht oder nur möglichst wenig herabsetzen.

Eine mögliche zusätzliche Komponente sind zum Beispiel Polymere, welche die Haftung des Mundbehandlungsmittels und die Benetzung mit diesem verbessern. Zu diesen Polymeren zählen Celluloseether, von denen Hydroxyethylcellulose, Hydroxypropylcellulose und/oder Hydroxymethylpropylcellulose bevorzugt sind. Auch Polyvinylpyrrolidone (PVP, Povidon, E 1201) sind geeignet, besonders solche mit einem Molekulargewicht von 15.000 bis 100.000. Geeignete Konzentrationen dieser Polymere liegen jeweils im Bereich von 0,01 bis 2 Gew.-%, insbesondere 0,05 bis 1 Gew.-%.

Das erfindungsgemäße Mittel zur Anwendung im Mund- und Rachenraum kann in jeder bereits bisher für derartige Mittel üblichen Darreichungsform konfektioniert werden. Eine bevorzugte Verwendung ist diejenige in Form wässriger Lösungen, die zum Spülen des Mund- und Rachenraums sowie zum Gurgeln eingesetzt werden. Die Anwendung in Form eines Sprays ist ebenso möglich. . Verfärbungen des Zahnfleisches, der Zunge oder der Zähne, wie sie im Fall von Chlorhexidin beobachtet werden, treten mit dem erfindungsgemäßen Mittel nicht auf. Das erfindungsgemäße Mittel ist zur Spülen des Mund- und Rachenbereichs zum Zweck der Beseitigung unangenehmer Gerüche und Ausdünstungen geeignet.

Nachfolgend sollen exemplarisch einige Beispiele für erfindungsgemäße Mittel zur Anwendung im Mund- und Rachenraum angegeben werden. Die angegebenen Zusammensetzungen sind jedoch rein beispielhaft zu verstehen, und die Erfindung soll in keiner Weise auf diese Beispiele beschränkt sein. Abwandlungen in der Art der verwendeten Komponenten und deren Konzentration sind im Rahmen der hier offenbarten Lehre ohne Weiteres möglich.

### Beispiel 1

Durch Mischen der nachfolgend angegebenen Komponenten wurde eine Mund- und Rachenspüllösung hergestellt.

| | | |
|---|---|---|
| Polyhexamethylenbiguanid (PHMB) | 0,1 | Gew.-% |
| Undecylensäureamidopropylbetain | 0,1 | Gew.-% |
| Natriumcyclamat | 0,25 | Gew.-% |
| Fukalyptusöl | 1,5 | Gew.-% |
| Macrogolglycerolhydroxylstearat (Cremophor® RH40) | 5 | Gew.-% |
| Gereinigtes Wasser | Rest | |

### Beispiel 2

Durch Mischen der nachfolgend angegebenen Komponenten wurde eine Mund- und Rachenspüllösung hergestellt.

| | | |
|---|---|---|
| Polyhexamethylenbiguanid (PHMB) | 0,1 | Gew.-% |
| Undecylensäureamidopropylbetain | 0,1 | Gew.-% |
| Natriumcyclamat | 0,2 | Gew.-% |
| Saccharin | 0,02 | Gew.-% |
| Orangenöl | 1,5 | Gew.-% |
| Macrogolglycerolhydroxylstearat (Cremophor® RH40) | 5 | Gew.-% |
| Hydroxyethylcellulose | 0,2 | Gew.-% |
| Gereinigtes Wasser | Rest | |

### Beispiel 3

Durch Mischen der nachfolgend angegebenen Komponenten wurde eine Mund- und Rachenspüllösung hergestellt.

| | | |
|---|---|---|
| Polyhexamethylenbiguanid (PHMB) | 0,1 | Gew.-% |
| Undecylensäureamidopropylbetain | 0,1 | Gew.-% |
| Natriumcyclamat | 0,1 | Gew.-% |
| Xylitol | 2,0 | Gew.-% |
| Thymianöl | 1,5 | Gew.-% |
| Macrogolglycerolhydroxylstearat (Cremophor® RH40) | 5 | Gew.-% |
| Gereinigtes Wasser | Rest | |

### Beispiel 4

Durch Mischen der nachfolgend angegebenen Komponenten wurde eine Mund- und Rachenspüllösung hergestellt.

| | | |
|---|---|---|
| Polyhexamethylenbiguanid (PHMB) | 0,1 | Gew.-% |
| Undecylensäureamidopropylbetain | 0,1 | Gew.-% |
| Natriumcyclamat | 0,1 | Gew.-% |
| Sorbitol | 3 | Gew.-% |
| Eukalyptusöl | 1,5 | Gew.-% |
| Macrogolglycerolhydroxylstearat (Cremophor® RH40) | 5 | Gew.-% |
| Gereinigtes Wasser | Rest | |

### Beispiel 5

Durch Mischen der nachfolgend angegebenen Komponenten wurde eine Mund- und Rachenspüllösung hergestellt.

| | | |
|---|---|---|
| Polyhexamethylenbiguanid (PHMB) | 0,1 | Gew.-% |
| Undecylensäureamidopropylbetain | 0,1 | Gew.% |
| Natriumcyclamat | 0,1 | Gew.-% |
| Glycerin | 4 | Gew.-% |
| Pfefferminzöl | 1,5 | Gew.-% |
| Macrogolglycerolhydroxylstearat (Cremophor® RH40) | 5 | Gew.-% |
| Hydroxyethylcellulose | 0,2 | Gew.-% |
| Gereinigtes Wasser | Rest | |

### Beispiel 6

Durch Mischen der nachfolgend angegebenen Komponenten wurde eine Mund- und Rachenspüllösung hergestellt.

| | | |
|---|---|---|
| Polyhexamethylenbiguanid (PHMB) | 0,1 | Gew.-% |
| Undecylensäureamidopropylbetain | 0,1 | Gew.-% |
| Natriumcyclamat | 0,1 | Gew.-% |
| Aspartam | 0,005 | Gew.-% |
| Acesulfam | 0,0025 | Gew.-% |
| Eukalyptusöl | 1,5 | Gew.-% |
| Macrogolglycerolhydroxylstearat (Cremophor® RH40) | 5 | Gew.-% |
| Gereinigtes Wasser | Rest | |

### Beispiel 7

Durch Mischen der nachfolgend angegebenen Komponenten wurde eine Mund- und Rachenspüllösung hergestellt.

| | | |
|---|---|---|
| Polyhexamethylenbiguanid (PHMB) | 0,1 | Gew.-% |
| Undecylensäureamidopropylbetain | 0,1 | Gew.-% |
| Xylitol | 2,0 | Gew.-% |
| Saccharin | 0,01 | Gew.-% |
| Eukalyptusöl | 1,5 | Gew.-% |
| Macrogolglycerolhydroxylstearat (Cremophor® RH40) | 5 | Gew.-% |
| Gereinigtes Wasser | Rest | |

### Beispiel 8

Durch Mischen der nachfolgend angegebenen Komponenten wurde eine Mund- und Rachenspüllösung hergestellt.

| | | |
|---|---|---|
| Polyhexamethylenbiguanid (PHMB) | 0,1 | Gew.-% |
| Undecylensäureamidopropylbetain | 0,1 | Gew.-% |
| Natriumcyclamat | 0,25 | Gew.-% |
| Pfefferminzöl | 1,5 | Gew.% |
| Polysorbat 80 | 5 | Gew.-% |
| Gereinigtes Wasser | Rest | |

## Patentansprüche

1. Mittel zur Anwendung im Mund- und Rachenraum, welches in wässriger Lösung als Mikrobizid ein lineares polymeres Biguanid und/oder ein wasserlösliches Salz desselben enthält,
**dadurch gekennzeichnet,**
**dass** es das Mikrobizid in Kombination mit wenigstens einem Süßstoff umfasst.

2. Mittel gemäß Anspruch 1,
**dadurchgekennzeichnet,**
**dass** es wenigstens einen Zuckeraustauschstoff umfasst.

3. Mittel gemäß Anspruch 1 oder 2,
**dadurch gekennzeichnet,**
**dass** das lineare polymere Biguanid Polyhexamethylenbiguanid ist.

4. Mittel gemäß einem der Ansprüche 1 bis 3,
**dadurch gekennzeichnet,**
**dass** der Süßstoff ausgewählt ist aus wenigstens einer der Gruppen Acesulfam, Aspartam, Cyclamat, Neohesperidin, Saccharin und Thaumatin, insbesondere Cyclamat, Neohesperidin, Saccharin und Thaumatin.

5. Mittel gemäß Anspruch 4,
**dadurch gekennzeichnet,**
**dass** der Süßstoff Cyclamat und insbesondere Natriumcyclamat umfasst.

6. Mittel gemäß Anspruch 5,
**dadurch gekennzeichnet,**
**dass** es Cyclamat allein oder in Kombination mit einem anderen Süßstoff und/oder Zuckeraustauschstoff enthält.

7. Mittel gemäß einem der Ansprüche 2 bis 5,
**dadurch gekennzeichnet,**
**dass** der Zuckeraustauschstoff ausgewählt ist aus Xylitol und/oder Sorbitol.

8. Mittel gemäß Anspruch 6 oder 7,
**dadurch gekennzeichnet,**
**dass** es Cyclamat in Kombination mit wenigstens einem Süßungsmittel aus der Gruppe Saccharin, Acesulfam, Aspartam, Xylitol und Sorbitol enthält.

9. Mittel gemäß Anspruch 8,
**dadurch gekennzeichnet,**
**dass** Cyclamat und weiteres Süßungsmittel in einem Gewichtsverhältnis von 1 : 100 bis 100 : 1 vorhanden sind.

10. Mittel gemäß einem der Ansprüche 1 bis 9,
**dadurch gekennzeichnet,**
**dass** es wenigstens ein Polyol enthält, insbesondere ausgewählt aus Glycerin, Propylenglykol und Polyethylenglykol, bevorzugt Polyethylenglykol mit einem Molekulargewicht von 400 bis 4000.

11. Mittel gemäß einem der Ansprüche 1 bis 10,
**dadurch gekennzeichnet,**
**dass** der Süßstoff in einer Konzentration von 0,01 bis 1 Gew.%, bevorzugt 0,02 bis 0,5 Gew.-% und insbesondere von 25 bis 2500 mg/l in der wässrigen Lösung enthalten ist.

12. Mittel gemäß einem der Ansprüche 2 bis 11,
**dadurch gekennzeichnet,**
**dass** der Zuckeraustauschstoff in einer Konzentration von 0,2 bis 10 Gew.-%, bevorzugt 1,0 bis 5,0 Gew.%, in der wässrigen Lösung enthalten ist.

13. Mittel gemäß einem der Ansprüche 1 bis 12,
**dadurchgekennzeichnet,**
**dass** es weiter wenigstens ein ätherisches Öl enthält, insbesondere ausgewählt aus wenigstens einem Öl der Gruppe Pfefferminz-, Eukalyptus-, Zitronen-, Orangen-, Grapefruit-, Thymian- und Kamillenöl.

14. Mittel gemäß Anspruch 13,
**dadurch gekennzeichnet,**
**dass** das ätherische Öl in einer Konzentration von 0,3 bis 3 Gew.%, bevorzugt 0,8 bis 3 Gew.%, in der wässrigen Lösung enthalten ist.

15. Mittel gemäß einem der Ansprüche 1 bis 14,
**dadurch gekennzeichnet,**
**dass** es weiter wenigstens ein Tensid, bevorzugt ein nicht-ionisches Tensid und/oder Amphotensid, besonders bevorzugt ein Glycin-Derivat und insbesondere ein Amidoalkylbetain einer Fettsäure, aufweist.

16. Mittel gemäß Anspruch 15,
**dadurch gekennzeichnet,**
**dass** das Glycin-Derivat ein Undecylenamidoalkylbetain, Cocamidoalkylbetain, Lauramidoalkylbetain oder Ricinolamidoalkylbetain ist, wobei der Alkylrest bevorzugt Ethyl oder Propyl ist.

17. Mittel gemäß Anspruch 16,
**dadurch gekennzeichnet,**
**dass** es Undecylensäureamidopropylbetain in einer Konzentration von 0,01 bis 1,5 Gew.%, insbesondere 0,03 bis 1 Gew.-% und bevorzugt 0,1 bis 0,4 Gew.-% enthält.

18. Mittel gemäß einem der Ansprüche 15 bis 17,
**dadurch gekennzeichnet,**
**dass** das Tensid ausgewählt ist aus einem Macrogol-Tensid und/oder Polysorbat, insbesondere aus Macrogolglycerolhydroxylalkylat, Macrogolalkylether, wobei Alkyl C₈₋₂₂- und insbesondere C₁₂₋₁₈-Alkyl ist, und/oder Polysorbat 80.

19. Mittel gemäß Anspruch 18,
**dadurch gekennzeichnet,**
**dass** das Tensid in einer Menge von 1,0 bis 10 Gew.-% in der wässrigen Lösung enthalten ist.

20. Mittel gemäß einem der Ansprüche 1 bis 19,
**dadurch gekennzeichnet,**
**dass** es weiter wenigstens einen Celluloseether, bevorzugt Hydroxyethylcellulose, Hydroxypropylcellulose und/oder Hydroxymethylpropylcellulose, und/oder ein Polyvinylpyrrolidon, bevorzugt mit einem Molekulargewicht von 15.000 bis 100.000, enthält.

21. Mittel gemäß einem der Ansprüche 3 bis 20,
**dadurch gekennzeichnet,**
**dass** es 0,01 bis 0,3 Gew.-% und bevorzugt 0,1 Ges.-% Polyhexamethylenbiguanid enthält.

22. Nicht-therapeutische Verwendung des Mittels gemäß einem der Ansprüche 1 bis 21 zur Spülung des Mund- und Rachenraums zur Beseitigung unangenehmer Gerüche und Ausdünstungen.

## Claims

1. A composition for application in the mouth and throat area, which contains a linear polymer biguanide and/or a water-soluble salt of the same in an aqueous solution as a microbiocide,
**characterized in that**
it comprises the microbiocide in combination with at least one sweetener.

2. A composition according to claim 1,
**characterized in that**
it comprises at least one sugar substitute.

3. A composition according to claim 1 or 2,
**characterized in that**
the linear polymer biguanide is polyhexamethylene biguanide.

4. A composition according to one of the claims 1 to 3,
**characterized in that**
the sweetener is chosen from at least one of the groups of acesulfame, aspartame, cyclamate, neohesperidin, saccharin and thaumatin, especially cyclamate, neohesperidin, saccharin and thaumatin.

5. A composition according to claim 4,
**characterized in that**
the sweetener comprises cyclamate and especially sodium cyclamate.

6. A composition according to claim 5,
**characterized in that**
it contains cyclamate alone or in combination, with another sweetener and/or sugar substitute.

7. A composition according to one of the claims 2 to 5,
**characterized in that**
the sugar substitute is chosen from xylitol and/or sorbitol.

8. A composition according to claim 6 or 7,
**characterized in that**
it contains cyclamate in combination with at least one sweetening agent from the group of saccharin, acesulfame, aspartame, xylitol and sorbitol.

9. A composition according to claim 8,
**characterized in that**
cyclamate and a further sweetening agent are present at a weight ratio of 1:100 to 100:1.

10. A composition according to one of the claims 1 to 9,
**characterized in that**
it contains at least one polyol, especially chosen from glycerine, propylene glycol and polyethylene glycol, preferably polyethylene glycol with a molecular weight of 400 to 4000.

11. A composition according to one of the claims 1 to 10,
**characterized in that**
the sweetener is contained in the aqueous solution in a concentration of 0.01 to 1 percent by weight, preferably 0.02 to 0.5 percent by weight, and especially 25 to 2500 mg/L.

12. A composition according to one of the claims 2 to 11,
**characterized in that**
the sugar substitute is contained in the aqueous solution in a concentration of 0.2 to 10 percent by weight, preferably 1.0 to 5.0 percent by weight.

13. A composition according to one of the claims 1 to 12,
**characterized in that**
it further contains at least one ethereal oil, especially chosen from at least one oil of the group of peppermint, eucalyptus, lemon, orange, grapefruit, thyme and chamomile oil.

14. A composition according to claim 13,
**characterized in that**
the ethereal oil is contained in the aqueous solution in a concentration of 0.3 to 3 percent by weight, preferably 0.8 to 3 percent by weight.

15. A composition according to one of the claims 1 to 14,
**characterized in that**
it further has at least one surfactant, preferably a non-ionic surfactant and/or amphoteric surfactant, especially preferably a glycine derivative and especially an amidoalkyl betaine of a fatty acid.

16. A composition according to claim 15,
**characterized in that**
the glycine derivative is an undecylene amidoalkyl betaine, cocamidoalkyl betaine, lauramidoalkyl betaine or ricinoleamidoalkyl betaine, with the alkyl residue preferably being ethyl or propyl.

17. A composition according to claim 16,
**characterized in that**
it contains undecylenic acid amidopropyl betaine in a concentration of 0.01 to 1.5 percent by weight, especially 0.03 to 1 percent by weight, and preferably 0.1 to 0.4 percent by weight.

18. A composition according to one of the claims 15 to 17,
**characterized in that**
the surfactant is chosen from a macrogol surfactant and/or polysorbat, especially from macrogol glycerol hydroxyalkylate, macrogol alkyl ether, with alkyl being C₈₋₂₂- and especially C₁₂₋₁₈ alkyl, and/or polysorbat 80.

19. A composition according to claim 18,
**characterized in that**
the surfactant is contained in a quantity of 1.0 to 10 percent by weight in the aqueous solution.

20. A composition according to one of the claims 1 to 19,
**characterized in that**
it further contains at least one cellulose ether, preferably hydroxyethyl cellulose, hydroxypropyl cellulose and/or hyroxymethyl propyl cellulose, and/or a polyvinyl pyrrolidone, preferably with a molecular weight of 15,000 to 100,000.

21. A composition according to one of the claims 3 to 20,
**characterized in that**
it contains 0.01 to 0.3 percent by weight and preferably 0.1 percent by weight of polyhexamethylene biguanide.

22. A non-therapeutic use of the composition according to one of the claims 1 to 21 for rinsing the mouth and throat area for the remedy of unpleasant smells and odors.

## Revendications

1. Produit pour l'application dans l'espace buccal ou pharyngé, qui contient en solution aqueuse, pour servir de microbicide, un biguanide polymère linéaire et/ou un sel hydrosoluble de celui-ci,
**caractérisé en ce qu'**il
contient le microbicide en combinaison avec au moins un édulcorant.

2. Produit selon la revendication 1,
**caractérisé en ce qu'**il
contient au moins un substitut de sucre.

3. Produit selon la revendication 1 ou 2,
**caractérisé en ce que**
le biguanide polymère linéaire est du polyhexaméthylène biguanide.

4. Produit selon l'une des revendications 1 à 3,
**caractérisé en ce que**
l'édulcorant est au moins une substance choisie parmi l'acésulfame, l'aspartame, le cyclamate, la néohespéridine, la saccharine et la thaumatine, en particulier le cyclamate, la néohespéridine, la saccharine et la thaumatine.

5. Produit selon la revendication 4,
**caractérisé en ce que**
l'édulcorant contient du cyclamate et en particulier du cyclamate de sodium.

6. Produit selon la revendication 5,
**caractérisé en ce qu'**il
contient du cyclamate seul ou en combinaison avec un autre édulcorant et/ou substitut de sucre.

7. Produit selon l'une des revendications 2 à 5,
**caractérisé en ce que** le
substitut de sucre est choisi parmi le xylitol et/ou le sorbitol.

8. Produit selon la revendication 6 ou 7,
**caractérisé en ce qu'**il
contient du cyclamate en combinaison avec au moins un édulcorant parmi le groupe comprenant la saccharine, l'acésulfame, l'aspartame, le xylitol et le sorbitol.

9. Produit selon la revendication 8,
**caractérisé en ce qu'**il
contient du cyclamate et un autre édulcorant dans un rapport de poids de 1 pour 100 à 100 pour 1.

10. Produit selon l'une des revendications 1 à 9,
**caractérisé en ce qu'**il
contient au moins un polyol, en particulier choisi parmi la glycérine, le propylène-glycol et le polyéthylène-glycol, de préférence du polyéthylène-glycol ayant un poids moléculaire de 400 à 4000.

11. Produit selon l'une des revendications 1 à 10,
**caractérisé en ce que**
l'édulcorant est présent à une concentration de 0,01 à 1 % en poids, de préférence de 0,02 à 0,5 % en poids et en particulier de 25 à 2500 mg/l dans la solution aqueuse.

12. Produit selon l'une des revendications 2 à 11,
**caractérisé en ce que**
le substitut de sucre est présent à une concentration de 0,2 à 10 % en poids, de préférence de 1,0 à 5,0 % en poids, dans la solution aqueuse.

13. Produit selon l'une des revendications 1 à 12,
**caractérisé en ce qu'**il
contient au moins une huile essentielle, choisie en particulier parmi au moins une essence du groupe des essences de menthe poivrée, d'eucalyptus, de citron, d'orange, de pamplemousse, de thym et de camomille.

14. Produit selon la revendication 13,
**caractérisé en ce que**
l'huile essentielle est présente à une concentration de 0,3 à 3 % en poids, de préférence de 0,8 à 3 % en poids, dans la solution aqueuse.

15. Produit selon l'une des revendications 1 à 14,
**caractérisé en ce qu'**il
contient au moins un tensioactif, de préférence un tensioactif non ionique et/ou un amphotère, en particulier un dérivé de glycine et notamment une amidoalkylbétaïne d'un acide gras.

16. Produit selon la revendication 15,
**caractérisé en ce que**
le dérivé de glycine est une amidoalkylbétaïne undécylénique, une amidoalkylbétaïne de noix de coco, une amidoalkylbétaïne laurique ou une amidoalkylbétaïne ricinolique, le radical alkyle étant de préférence un éthyle ou un propyle.

17. Produit selon la revendication 16,
**caractérisé en ce qu'**il
contient de l'amidopropylbétaïne d'acide undécylénique à une concentration de 0,01 à 1,5 % en poids, de préférence de 0,03 à 1 % en poids et en particulier de 0,1 à 0,4 % en poids.

18. Produit selon l'une des revendications 15 à 17,
**caractérisé en ce que**
le tensioactif est choisi parmi un macrogol et/ou un polysorbate, en particulier parmi l'hydroxyalkylate de macrogol-glycérol, l'éther alkylique de macrogol, l'alkyle étant un alkyle en C₈-C₂₂ et en particulier un alkyle en C₁₂-C₁₈, et/ou du Polysorbate 80.

19. Produit selon la revendication 18,
**caractérisé en ce que**
le tensioactif est présent à raison de 1,0 à 10 % en poids dans la solution aqueuse.

20. Produit selon l'une des revendications 1 à 19,
**caractérisé en ce qu'**il
contient en outre au moins un éther de cellulose, de préférence de l'hydroxyéthylcellulose, de l'hydroxypropylcellulose et/ou de l'hydroxyméthylpropylcellulose, et/ou une polyvinylpyrrolidone, ayant de préférence un poids moléculaire de 15 000 à 100 000.

21. Produit selon l'une des revendications 3 à 20,
**caractérisé en ce qu'**il
contient 0,01 à 0,3 % en poids et de préférence 0,1 % en poids de polyhexaméthylène biguanide.

22. Utilisation non thérapeutique du produit selon l'une des revendications 1 à 21 pour le rinçage de l'espace bucco-pharyngé en vue d'éliminer les odeurs et émanations désagréables.
